# EUROPEAN PATENT SPECIFICATION

(11) **EP 1 704 845 B1**
(45) Date of publication and mention of the grant of the patent: **03.07.2019**
(21) Application number: 05703950.5
(22) Date of filing: 14.01.2005
(51) Int. Cl.: C08F 4/52, A61K 6/00, A61L 24/00, C09J 4/00

(54) **Polymerization initiator composition for dental or surgical adhesive compositions**
Polymerisationsinitiatorzusammensetzung für zahnärztliche oder chirurgische Klebstoffzusammensetzungen
Composition d'initiateur de polymérisation pour compositions adhésives dentaires ou chirurgicales

(30) Priority: 15.01.2004 JP 2004007815
(43) Date of publication of application: 27.09.2006
(73) Proprietor: SUN MEDICAL CO., LTD., Shiga 524-0044 (JP)
(72) Inventor: ZENG, Weiping, c/o Sun Medical Co., Ltd., Moriyama-shi, Shiga 524-0044 (JP); MUSOU, Haruki, c/o Sun Medical Co., Ltd., Moriyama-shi, Shiga 524-0044 (JP)
(74) Representative: Beckmann, Claus
(86) International application number: PCT/JP2005/000722
(87) International publication number: WO 2005/067866

(56) References cited:
- EP-A1- 0 567 213
- EP-A2- 0 758 544
- JP-A- 5 253 284
- JP-A- 9 110 913
- JP-A- 9 309 811
- US-A- 5 122 061

## Description

### TECHNICAL FIELD

The present invention relates to an adhesive composition for dental or surgical use and a polymerization initiator composition for the same. More specifically, it relates to an adhesive composition for dental or surgical use which has high curability and adhesion and a polymerization initiator composition for use in the same, which has improved safety against ignition when it adheres to paper or the like.

### BACKGROUND ART

JP-B 51-37092 discloses an adhesive for dental or surgical use which comprises a partially oxidated trialkyl boron as a product obtained by reacting 0.3 to 0.9 mole of oxygen with a trialkyl boron as a polymerization initiator. Since the trialkyl boron is very unstable in air and quickly reacts with oxygen to ignite when it is exposed to air as disclosed by JP-B 51-37092, it is very dangerous in handling. JP-B 51-37092 proposes a polymerization initiator having improved safety against ignition by suppressing a reduction in the activity of the trialkyl boron as much as possible but could not suppress its ignitability completely.

JP-A 5-253284 proposes a polymerization initiator having improved safety by adding a polar organic compound or an inert diluent as for the ignitability of a trialkyl boron. However, it cannot be said that the polymerization initiator was satisfactory.

JP-A 48-11892 discloses a method of preparing a paste by adding a viscous substance having hydrophobic nature such as Vaseline, paraffin or silicone, that is, silicon oil and optionally an adsorbent such as silicic acid or alumina to a trialkyl boron or a derivative thereof so as to improve its safety against ignition. JP-B 3-54683 proposes a polymerization initiator as a homogeneous mixture which is prepared by adding an organic oligomer or an organic polymer such as silicon oil, wax, oligoester or oligoamide to an organic boron compound.

JP-A 3-264509 proposes a method of preparing a paste by adding a polymer of an alkyl (meth)acrylate to tributyl boron or partially oxidated tributyl boron so as to improve its safety against ignition. A proposal for converting the butyl group of tributyl boron into a structure that hardly ignites in air is disclosed by EP-51797, JP-A 3-70753, West German Patent No. 3201731, JP-A 5-253284 and JP-B 6-8301.

JP-A 9-110913 proposes a method of improving safety by adding an aprotic solvent and further an inert liquid or solid organic oligomer or polymer to tributyl boron or partially oxidated tributyl boron. Addition of a large amount of the additive tends to reduce the activity of the polymerization initiator, and the viscosity of the initiator composition is increased by the addition of the organic oligomer or polymer, whereby it may be difficult to collect an exact amount of the initiator composition.

### DISCLOSURE OF THE INVENTION

It is an object of the present invention to provide a polymerization initiator composition which does not burn or ignite even when it contacts paper or the like in air and can provide high polymerization activity to a polymerizable composition to cure it in a short period of time.

It is another object of the present invention to provide a polymerization initiator composition suitable for use in an adhesive composition for dental or surgical use, particularly for dental use, which can be collected in an exact amount even though the amount per each time of use is very small, for example, several mg or several tens milligrams, and has high fluidity.

It is still another object of the present invention to provide an adhesive composition for dental or surgical use, which comprises the above polymerization initiator composition of the present invention and exhibits high adhesion.

Other objects and advantages of the present invention will become apparent from the following description.

According to the present invention, firstly, the above objects and advantages of the present invention are attained by a polymerization initiator composition comprising 100 parts by weight of (A) at least one organic boron compound selected from the group consisting of a trialkyl boron, alkoxyalkyl boron, dialkyl borane and partially oxidated trialkyl boron, 5 to 40 parts by weight of (B) an aprotic solvent having a boiling point of 50 to 120°C, and 0.2 to 5 parts by weight of (C) an alcohol having a boiling point of 60 to 180°C.

According to the present invention, secondly, the above objects and advantages of the present invention are attained by an adhesive composition for dental or surgical use, comprising 30 to 100 parts by weight of (a) a polymerizable monomer, 0 to 70 parts by weight of (b) a (meth)acrylate polymer and 1 to 20 parts by weight of (c) the above polymerization initiator composition of the present invention, the total of the polymerizable monomer (a) and the (meth)acrylate polymer (b) being 100 parts by weight.

### BEST MODE FOR CARRYING OUT THE INVENTION

The organic boron compound (A) used in the present invention is at least one organic boron compound selected from the group consisting of a trialkyl boron, alkoxyalkyl boron, dialkyl borane and partially oxidated trialkyl boron. They may be used alone or in combination of two or more.

The trialkyl boron preferably has linear, branched or cyclo-cyclic alkyl groups having 2 to 8 carbon atoms. Examples of the trialkyl boron include triethyl boron, tripropyl boron, triisopropyl boron, tributyl boron, tri-sec-butyl boron, triisobutyl boron, tripentyl boron, trihexyl boron, triheptyl boron, trioctyl boron, tricyclopentyl boron and tricyclohexyl boron. As described above, the three alkyl groups may be the same, only two out of the three alkyl groups may be same, or the three alkyl groups may be different. In the last case, the alkyl groups of the above trialkyl borons may be suitably combined.

The alkoxyalkyl boron is, for example, a monoalkoxydialkyl boron or a dialkoxymonoalkoxyl boron. The alkoxy group and alkyl group are linear, branched or cyclo-cyclic alkyl groups having 2 to 8 carbon atoms. Examples of the monoalkoxydialkyl boron include butoxydibutyl boron. It is preferred that the alkyl moiety of the alkoxy group and the alkyl group should be the same like the above example because raw materials are easily acquired. However, they are not particularly limited. Examples of the dialkyl borane include dicyclohexyl borane and diisoamyl borane. The two alkyl groups may be the same like these examples or different as the case may be. A monocyclo or bicyclo compound having a boron atom as a hetero atom, such as 9-borabicyclo[3.3.1]nonane is also included.

The partially oxidated trialkyl boron is, for example, a partial oxide of the above trialkyl boron. Partially oxidated tributyl boron is particularly preferred. A partially oxidated trialkyl boron obtained by adding preferably 0.3 to 0.9 mole, more preferably 0.4 to 0.6 mole of oxygen to 1 mole of a trialkyl boron is used as the partially oxidated trialkyl boron.

When tributyl boron or partially oxidated tributyl boron is used out of these organic boron compounds, particularly good results are obtained. The most preferred organic boron compound is partially oxidated tributyl boron. As described above, the three alkyl groups may be the same, only two out of the three alkyl groups may be same, or the three alkyl groups may be different.

The aprotic solvent (B) is preferably a solvent having a boiling point of 50 to 120° C at 1 atm. The boiling point is more preferably 60 to 90° C. When the boiling point of the aprotic solvent (B) is lower than the above lower limit, the aprotic solvent (B) volatilizes and scatters during the conveyance and storage of the polymerization initiator of the present invention, whereby its content decreases and its effect of suppressing ignition lowers disadvantageously. When the boiling point of the aprotic solvent is higher than the above upper limit, its volatilization and scattering during use become unsatisfactory, whereby it remains in the cured composition and reduces the adhesion of the adhesive composition disadvantageously.

The aprotic solvent (B) is preferably a solvent which does not have active hydrogen as existent in the hydroxyl group or mercapto group reactive with the organic boron compound (A) and can form a homogeneous solution with the organic boron compound (A).

Specific examples of the aprotic solvent (B) include hydrocarbons such as pentane, hexane, cyclohexane, heptane, benzene and toluene; halogenated hydrocarbons such as fluorobenzene, dichloroethane and so-called "furon"; ethers such as diethyl ether, diisopropyl ether, ethylene glycol dimethyl ether and tetrahydrofuran; ketones such as acetone, methyl ethyl ketone and diethyl ketone; and esters such as methyl acetate, ethyl acetate and isopropyl acetate. Out of these, alkanes, ethers and esters are preferred, and hexane, diisopropyl ether and ethyl acetate are particularly preferred. These aprotic solvents (B) may be used alone or in combination of two or more.

The content of the aprotic solvent (B) in the polymerization initiator composition of the present invention must be 5 to 40 parts by weight, preferably 10 to 30 parts by weight, most preferably 10 to 25 parts by weight based on 100 parts by weight of the organic boron compound (A). When the content of the aprotic solvent (B) is lower than 5 parts by weight based on 100 parts by weight of the organic boron compound (A), a satisfactory dilution effect is not obtained and the effect of suppressing heat generation (ignition) is not obtained. When the content of the aprotic solvent (B) is higher than 40 parts by weight based on 100 parts by weight of the organic boron compound (A), it tends to reduce the polymerizability of the polymerization initiator composition.

Surprisingly, the alcohol (C) in the initiator composition of the present invention suppresses heat generation (ignition) without reducing polymerization activity in the presence of the aprotic solvent (B) when it is added in a specific small amount. The alcohol (C) has a boiling point of 60 to 180° C at 1 atm. The preferred boiling point is 60 to 120° C. When the boiling point of the alcohol (C) is lower than the above lower limit, the alcohol (C) volatilizes and scatters during the conveyance and storage of the polymerization initiator of the present invention, whereby its content decreases and its effect of suppressing ignition lowers disadvantageously. When the boiling point of the alcohol is higher than the above upper limit, the curing time of the adhesive composition becomes long and the adhesion of the adhesive composition degrades disadvantageously.

Examples of the alcohol (C) include methanol, ethanol, n-propanol and isomers thereof, n-butanol and isomers thereof, n-pentanol and isomers thereof, n-hexanol and isomers thereof, and n-heptanol and isomers thereof. It is more preferably an alcohol having 4 or less carbon atoms, particularly preferably ethanol or n-propanol. These alcohols (C) may be used alone or in combination of two or more.

The content of the alcohol (C) in the polymerization initiator composition of the present invention must be 0.2 to 5 parts by weight, preferably 0.3 to 4.5 parts by weight, most preferably 0.5 to 4 parts by weight based on 100 parts by weight of the organic boron compound (A). When the content of the alcohol (C) is lower than 0.2 part by weight based on 100 parts by weight of the organic boron compound (A), its effect of suppressing heat generation (ignition) is not fully obtained. When the content of the alcohol (C) is higher than 5 parts by weight based on 100 parts by weight of the organic boron compound (A), it tends to reduce the polymerizability of the polymerization initiator composition.

According to the present invention, there is further provided an adhesive composition for dental use, comprising the above polymerization initiator composition of the present invention.

That is, according to the present invention, there is further provided an adhesive composition for dental or surgical use which comprises preferably 30 to 100 parts by weight, more preferably 40 to 80 parts by weight of (a) a polymerizable monomer, preferably 0 to 70 parts by weight, more preferably 10 to 60 parts by weight of (b) a (meth)acrylate polymer, and preferably 1 to 20 parts by weight, more preferably 3 to 15 parts by weight of (c) the above polymerization initiator composition of the present invention, the total of the polymerizable monomer (a) and the (meth)acrylate polymer (b) being 100 parts by weight. When the amount of the above component (a) is smaller than the above lower limit, the operation efficiency of the composition lowers disadvantageously. When the amount of the above component (b) is larger than the above upper limit, the operation efficiency of the composition lowers disadvantageously. When the amount of the component (c) is smaller than the above lower limit, the curing time of the adhesive composition becomes long disadvantageously. When the amount of the component (c) is larger than the above upper limit, the curing time of the adhesive composition becomes too short, thereby impairing the operation efficiency disadvantageously. The term "(meth)acrylate" means acrylate and/or methacrylate. For example, "methyl (meth)acrylate" means methyl acrylate and/or methyl methacrylate. It should be understood that this term is idiomatically used in this technical field.

In the adhesive composition of the present invention, a known monofunctional monomer or polyfunctional monomer may be used as the above polymerizable monomer (a) without restrictions. A (meth) acrylate-based monomer is preferably used because it has relatively low irritant action to the human body. A polymerizable monomer having an acid group in the molecule is preferred as a component providing high adhesion to the dentine in particular. Therefore, a combination of a (meth)acrylate and a polymerizable monomer having an acid group is also preferably used.

Examples of the monofunctional (meth) acrylate include alkyl (meth)acrylates such as methyl (meth)acrylate, ethyl (meth)acrylate, propyl (meth)acrylate, butyl (meth)acrylate, hexyl (meth)acrylate, 2-ethylhexyl (meth)acrylate, dodecyl (meth)acrylate, lauryl (meth)acrylate, cyclohexyl (meth)acrylate, benzyl (meth)acrylate and isobornyl (meth)acrylate; hydroxylakyl (meth)acrylates such as 2-hydroxyethyl (meth)acrylate, 2- or 3-hydroxypropyl (meth)acrylate, 4-hydroxybutyl (meth)acrylate, 5-hydroxypentyl (meth)acrylate, 6-hydroxyhexyl (meth)acrylate, 1,2- or 1,3-dihydroxypropyl mono(meth)acrylate and erythritol mono(meth)acrylate; polyethylene glycol mono(meth)acrylates such as diethylene glycol mono(meth)acrylate, triethylene glycol mono(meth)acrylate, polyethylene glycol mono(meth)acrylate and polypropylene glycol mono(meth)acrylate; (poly)glycol monoalkyl ether (meth)acrylates such as ethylene glycol monomethyl ether (meth)acrylate, ethylene glycol monoethyl ether (meth)acrylate, diethylene glycol monomethyl ether (meth)acrylate, triethylene glycol monomethyl ether (meth)acrylate, polyethylene glycol monomethyl ether (meth)acrylate and polypropylene glycol monoalkyl ether (meth)acrylate; fluoroalkyl (meth)acrylates such as perfluorooctyl (meth)acrylate and hexafluorobutyl (meth)acrylate; silane compounds having a (meth)acryloxyalkyl group such as γ-(meth)acryloxypropyltrimethoxysilane and γ-(meth)acryloxypropyltri(trimethylsiloxy)silane; and (meth)acrylates having a hetero ring such as tetrafurfuryl (meth)acrylate.

Examples of the polyfunctional (meth)acrylate include alkane polyol poly(meth)acrylates such as ethylene glycol di(meth)acrylate, propylene glycol di(meth)acrylate, butylenes glycol di(meth)acrylate, neopentyl glycol di(meth)acrylate, hexylene glycol di(meth)acrylate, 2-hydroxypropyl di(meth)acrylate, trimethylolpropane tri(meth)acrylate and pentaeryhtritol tetra(meth)acrylate; polyoxyalkane polyol poly (meth) acrylates such as diethylene glycol di(meth)acrylate, triethylene glycol di(meth)acrylate, polyethylene glycol di(meth)acrylate, dipropylene glycol di(meth)acrylate, polypropylene glycol di(meth)acrylate, dibutylene glycol di(meth)acrylate and dipentaerythritol hexa(meth)acrylate; aliphatic and aromatic di(meth)acrylates represented by the following formula (1): 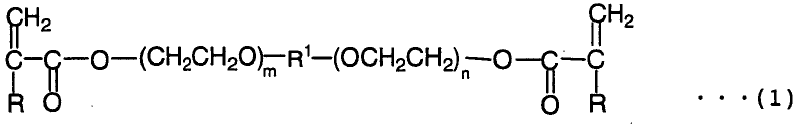 wherein R is a hydrogen atom or methyl group, m and n are the same or different and each an integer of 0 to 10, R¹ is 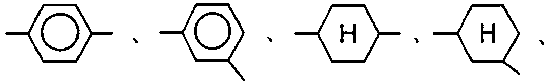 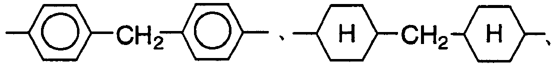 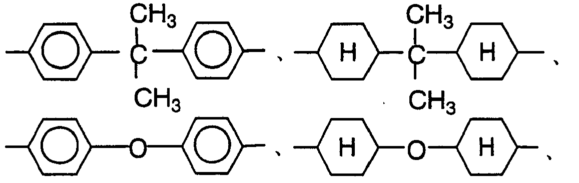 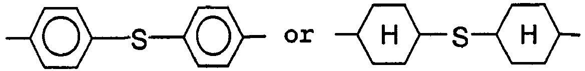 ; aliphatic and aromatic di(meth)acrylates having a hydroxyl group in the molecule and represented by the following formula (2): 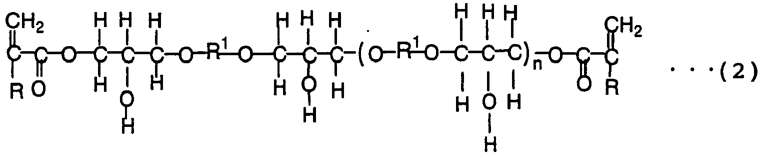 wherein R is a hydrogen atom or methyl group, n is an integer of 0 to 10, and R¹ is 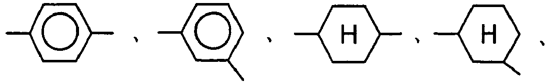 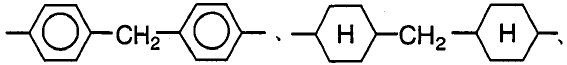 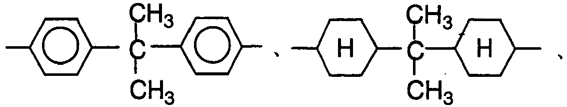 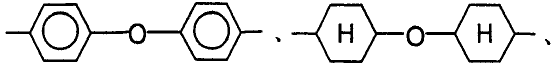 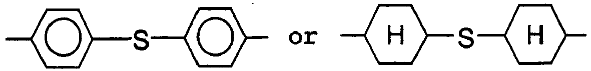 ; and polyfunctional (meth)acrylates having an urethane bond in the molecule and represented by the following formula (3) : 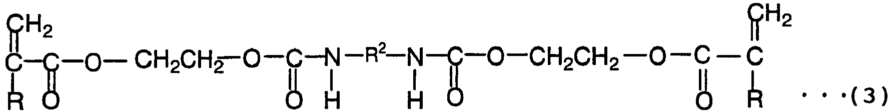 wherein R is a hydrogen atom or methyl group and R² is

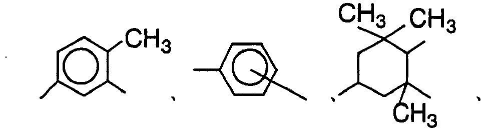

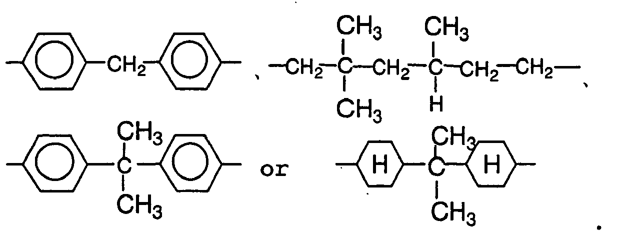

Out of these, alkyl (meth)acrylates such as methyl (meth)acrylate and ethyl (meth)acrylate; (meth)acrylates having a hydroxyl group such as 2-hydroxyethyl (meth)acrylate, 1, 3-dihydroxypropyl mono (meth)acrylate and erythritol mono(meth)acrylate; and (meth)acrylates having an ethylene glycol chain in the molecule such as triethylene glycol monomethyl ether (meth)acrylate and triethylene glycol mono(meth)acrylate are particularly preferred as the monofunctional (meth)acrylate.

Di(meth)acrylates having an ethylene glycol chain in the molecule such as triethylene glycol di(meth)acrylate and polyethylene glycol di(meth)acrylate, 2-hydroxypropyl di(meth)acrylate, compounds represented by the following formula (1)-a: 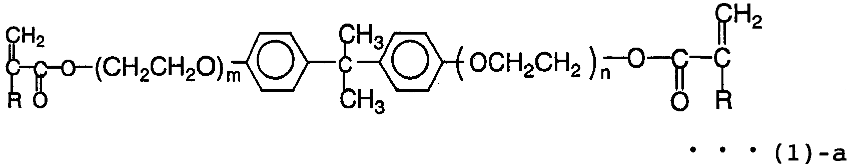 wherein R, m and n are as defined in the above formula (1), compounds represented by the following formula (2)-a: 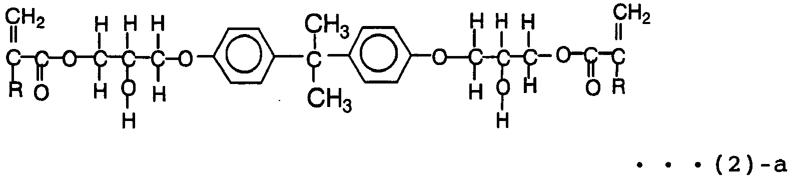 wherein R is as defined in the above formula (2) and compounds represented by the formula (3)-a: 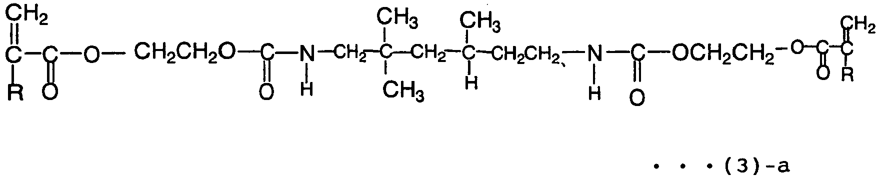 wherein R is as defined in the above formula (3) are particularly preferred as the polyfunctional (meth)acrylate. They may be used alone or in combination of two or more.

Examples of the polymerizable monomer having an acid group in the molecule include monomers having a carboxylic acid group or anhydride thereof such as (meth)acrylic acid and anhydride thereof, 1,4-di(meth)acryloxyethylpyromellitic acid, 6-(meth)acryloxyethylnaphthalene-1,2,6-tricarboxylic acid, N-(meth)acryloyl-p-aminobenzoic acid, N-(meth)acryloyl-o-aminobenzoic acid, N-(meth)acryloyl-m-aminobenzoic acid, N-(meth)acryloyl-5-aminosalicylic acid, N-(meth)acryloyl-4-aminosalicylic acid, 4-(meth)acryloxyethyltrimellitic acid and anhydride thereof, 4-(meth)acryloxybutyltrimellitic acid and anhydride thereof, 4-(meth)acryloxyhexyltrimellitic acid and anhydride thereof, 4-(meth)acryloxydecyltrimellitic acid and anhydride thereof, 2-(meth)acryloyloxybenzoic acid, 3-(meth)acryloyloxybenzoic acid, 4-(meth)acryloyloxybenzoic acid, β-(meth)acryloyloxyethylhydrogen succinate, β-(meth)acryloyloxyethylhydrogen maleate, β-(meth)acryloyloxyethylhydrogen phthalate, 11-(meth)acryloyloxy-1,1-undecanedicarboxylic acid and p-vinylbenzoic acid; monomers containing a phosphoric acid group such as (2-(meth)acryloxyethyl)phosphoric acid, (2-(meth)acryloxyethylphenyl)phosphoric acid and 10-(meth)acryloxydecylphosphoric acid; and monomers containing a sulfonic acid group such as p-styrenesulfonic acid and 2-acrylamido-2-methylpropanesulfonic acid.

These acid monomers may be used alone or in combination. The amount of the polymerizable monomer having an acid group is preferably 2 to 20 parts by weight based on 100 parts by weight of the total of the polymerizable monomers (a).

The (meth)acrylate polymer (b) contained in the adhesive composition of the present invention is, for example, a homopolymer of an alkyl (meth)acrylate, a copolymer of alkyl (meth)acrylates, a copolymer of an alkyl (meth)acrylate and another polymerizable monomer, a copolymer of an alkyl (meth)acrylate and an alkylene di(meth)acrylate, or a copolymer of an alkyl (meth)acrylate and a diene monomer. They may be used alone or in combination of two or more. Specific examples of the (meth)acrylate polymer (b) include non-crosslinkable polymers such as polymethyl (meth)acrylate, polyethyl (meth)acrylate, methyl (meth)acrylate·ethyl (meth)acrylate copolymer, methyl (meth)acrylate·butyl (meth)acrylate copolymer and methyl (meth)acrylate·styrene copolymer; and crosslinkable polymers such as methyl (meth)acrylate·ethylene glycol di(meth)acrylate copolymer, methyl (meth)acrylate·triethylene glycol di(meth)acrylate copolymer and a copolymer of methyl (meth)acrylate and a budiene-based monomer. The component (b) may be used as an inorganic particle of a metal oxide or a metal salt coated with the above alkyl methacrylate-based polymer. The component (b) may be pre-mixed with the component (a).

The adhesive composition of the present invention may be mixed with suitable amounts of an inorganic or organic filler; an organic composite filler; a filler colorant; and a polymerization inhibitor such as a hydroquinone as required.

Examples of the inorganic filler include metal oxide powders such as zirconium oxide, bismuth oxide, titanium oxide, zinc oxide and aluminum oxide particle, metal salt powders such as calcium carbonate, bismuth carbonate, calcium phosphate, zirconium phosphate and barium sulfate, glass fillers such as silica glass, aluminum-containing glass, barium-containing glass, strontium-containing glass and zirconium silicate glass, fillers having a silver releasing function, and fillers having a fluorine releasing function. These inorganic fillers may be used alone or in combination.

To obtain firm bonding between the inorganic filler and the resin, an inorganic filler subjected to a surface treatment such as silane treatment and polymer coating is preferably used.

To improve the effect of reducing the thickness of a film of the adhesive composition and repairing the film, the average particle diameter of the above particles is preferably in the range of 0.01 to 30 µm, particularly preferably in the range of 0.05 to 25 µm.

The compositions and blending of the adhesive for dental use and the polymerization initiator composition of the present invention are formulated, and a chemical reaction may occur by mixing and produce a product different from the above product. For example, it is possible that a trialkyl boron which is an organic boron compound may be reacted with an alcohol to produce an alkoxyalkyl boron.

The adhesive composition of the present invention is used for dental or surgical purpose. Prior to the use of the adhesive composition for dental purpose, a tooth is preferably pretreated. The pretreatment is, for example, the etching of a mating surface by an acid solution, the modification of the mating surface by a primer, or the etching and modification of the mating surface by a primer having etching capability. The acid solution used for etching is an aqueous solution containing 5 to 60 wt% of phosphoric acid, or an aqueous solution containing 10 wt% of citric acid and 3 wt% of ferric chloride. The primer used for the modification of the mating surface is preferably an aqueous solution containing 20 to 50 wt% of 2-hydroxyethyl acrylate, 2-hydroxymethyl acrylate or 1,3-dihydroxypropyl mono(meth)acrylate. The primer having etching capability used for the etching and modification of the mating surface is preferably an aqueous solution containing an organic acid (including a monomer having an acid group) and a component for modifying the delimed dentine and promoting the diffusion of the adhesive composition into the dentine. Examples of the component for promoting the diffusion of the adhesive composition into the dentine include hydroxyl group-containing monomers such as alkylene glycol, polyalkylene glycol, 2-hydroxyethyl (meth)acrylate and 1,3-dihydroxypropyl mono(meth)acrylate, and polyethylene glycol (meth)acrylate.

The initiator and the adhesive composition comprising the same of the present invention have high affinity for living organisms and are suitable for bond repairing a living organism such as bond repairing a tooth, protecting the wound of a soft organism, or bond fixing at the time of a surgical treatment.

### Examples

The following examples are provided for the purpose of further illustrating the present invention but are in no way to be taken as limiting. The following abbreviations denote respective compounds below.
TBB: tributyl boron (of Sun Medical Co., Ltd.)
TBB·O: partially oxidated tributyl boron (of Sun Medical Co. , Ltd., addition of 0.5 mole of oxygen to 1 mole of tributyl boron)
MMA: methyl methacrylate (of Wako Pure Chemical Industries, Ltd., special grade)
4-META: 4-methacryloxyethyl trimellitic anhydride (of Sun Medical Co., Ltd.)
p-(MMA/BuMA): copolymer of methyl methacrylate and butyl methacrylate (number average molecular weight of 120,000, particle diameter of 68 µm, MMA content of about 25 wt%, manufactured by Fujikura Kasei Co., Ltd.)
p-MMA: polymethyl methacrylate powder (number average molecular weight of 400,000, average particle diameter of about 25 µm, manufactured by Sun Medical Co., Ltd.)

### Examples 1 to 6 and Comparative Examples 1 to 12

### (1) preparation of polymerization initiator composition

Predetermined amounts of TBB·O (TBB only in Comparative Example 2) and a solvent shown in Table 1 were fed to a conical flask with a stopper and a Teflon stirrer in a nitrogen box and stirred for 10 hours. The obtained polymerization initiator composition was charged into a syringe having a capacity of 1 ml and a needle inner diameter of 0.3 mm in a nitrogen atmosphere.

### (2) properties of polymerization initiator composition

Tests were made on Examples 1 to 6 and Comparative Examples 1 to 12 according to compositions shown in Table 1. The results are shown in Table 1. The tests were conducted as follows.

### Ignition test:

0.5 ml (test 1) of the polymerization initiator composition was dropped on filter paper (Whatman, No.3) or 5 droplets (about 0.03 g, test 2) of the above composition were let fall on 8 sheets of facial tissue paper at 23±2°C to check its ignition, burning or fuming.

### Droplet collectability test:

The polymerization initiator composition was dropped from the syringe to judge the dropping state of the composition visually.
Excellent droplet fluidity: Each droplet falls and no stringing and no entry of air bubbles are seen,
poor droplet fluidity: A chain of droplets fall, or viscous stringing or entry of air bubbles is seen.
Polymerization activity test (measurement of curing time):
(1) 8 droplets (0.18 g) of a polymerizable monomer consisting of MMA and 4-META in a weight ratio of 95/5 and 2 droplets (0.011 to 0.014 g) of a polymerization initiator composition were collected in a dispensing dish at 23±2°C and 0.16 g of p-MMA was added to be mixed with the above mixture gently for 10 seconds to prepare a resin suspension.
(2) Vaseline was thinly coated on a glass sheet, a Teflon ring (outer diameter of 13 mm, inner diameter of 10 mm, thickness of 5 mm) thinly coated with Vaseline likewise was placed on the glass sheet, and the above resin suspension was poured into the obtained container.
(3) Within 30 seconds after the start of mixing, the above resin suspension was transferred to an incubator set to 37±2° C and a humidity of 100 %, a Vicat needle was dropped quietly on the surface of the specimen to check if a mark of the needle was left on the surface. The time duration from the start of mixing to the time when the mark disappeared from the specimen was taken as curing time.

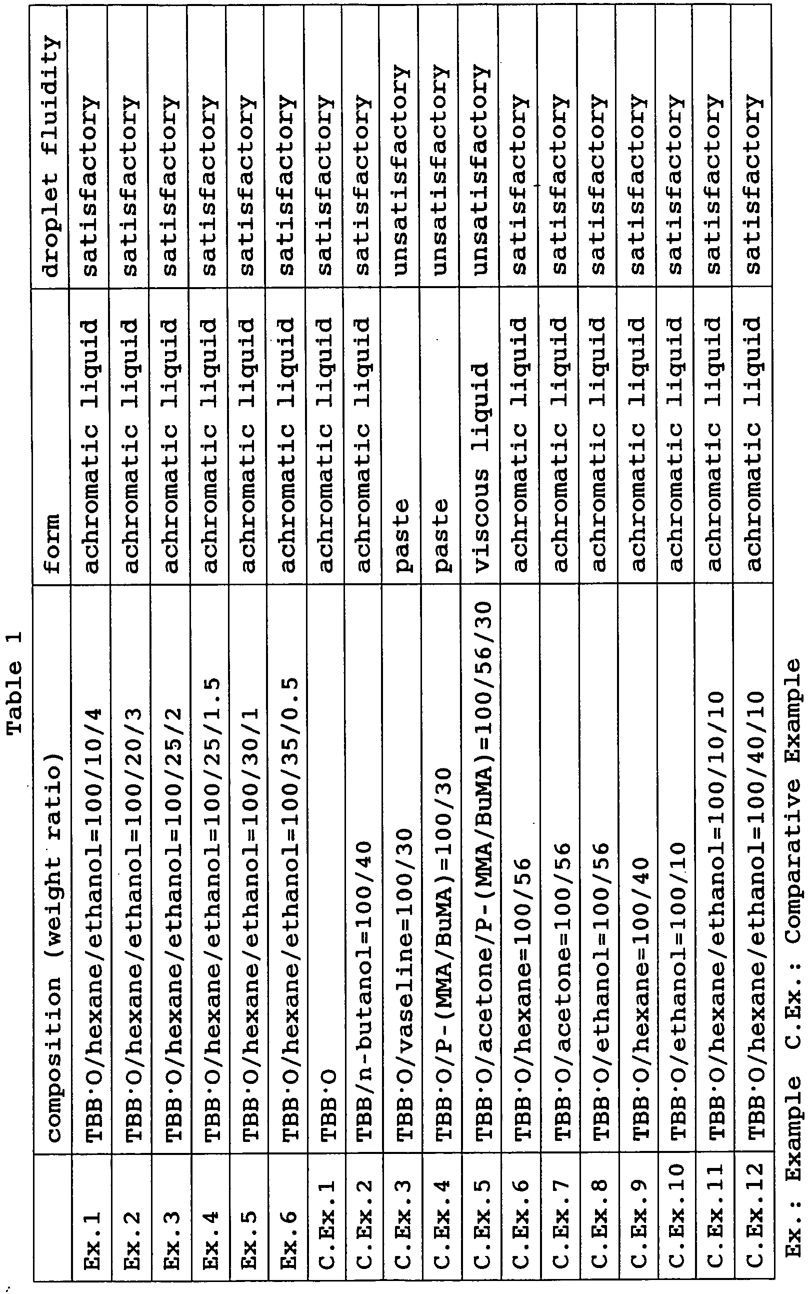

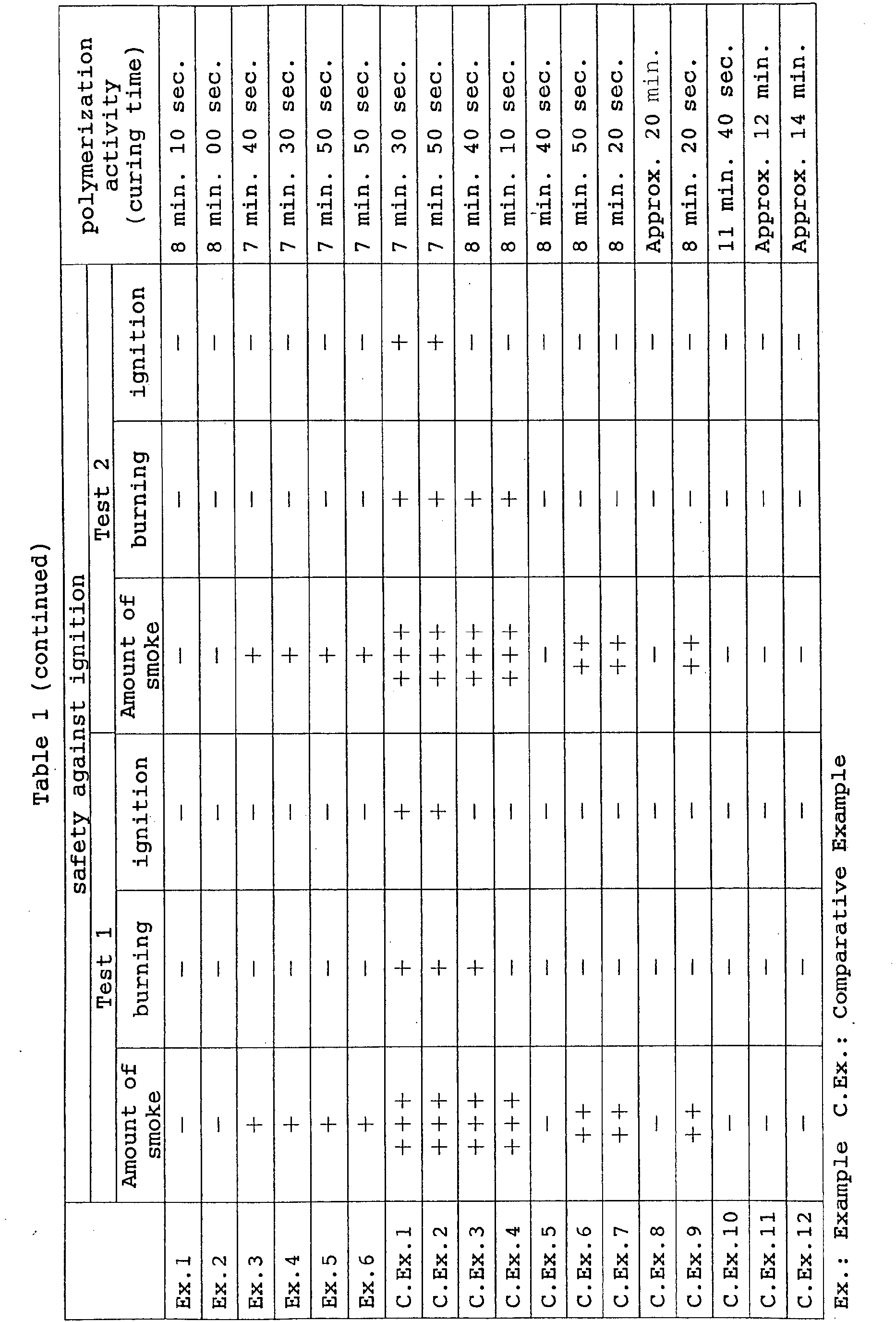

Explanations of symbols in the column for test on safety against ignition in the table above
No fuming/burning/ignition: -
Burnt/ignited: +
Amount of smoke Large: +++
   Slightly large: ++
   Small: +

### Example 7

(1) The lip side of a bovine foretooth was cut under running water to obtain a dentine which was then polished with No. 600 Emery paper so as to form a mating surface. This mating surface was dried, treated with an etching solution containing 10 wt% of citric acid and 3 % of iron chloride for 10 seconds, rinsed for 10 seconds and dried with air for 15 seconds. A cellophane tape having a 4 mm-diameter round hole was affixed to the mating surface to define an adhesion area.
(2) 4 droplets (0.09 g) of a polymerizable monomer consisting of MMA and 4-META in a weight ratio of 95/5 and one droplet (0.007 g) of the polymerization initiator composition of Example 3 were collected in a dispensing dish, and 0.07 g of p-MMA and 0.07 g of ZrO₂ fine particles (average particle diameter of about 5 µm, surface coated with about 3 wt% of PMMA) were added to and mixed with the above mixture gently for about 10 seconds to prepare a resin suspension.
(3) This resin suspension was applied to the mating surface prepared in (1) and an acrylic bar is bonded to the mating surface to prepare an adhesion test sample.

This adhesion test sample was left at room temperature for 30 minutes and immersed in distilled water at 37°C for 24 hours, and a tensile test was made onto this sample to measure adhesive strength between the acrylic bar and the dentine. The adhesive strength is the average of 5 measurement values of the sample. The adhesive strength was 13.5 MPa.

As described above, according to the present invention, an organic boron-based polymerization initiator composition which is excellent in safety against ignition, fluidity and polymerization activity can be obtained and further an adhesive composition for dental or surgical use which is excellent in safety, operation efficiency, adhesion and economy is obtained by using the above composition.

## Claims

1. A polymerization initiator composition for a polymerizable monomer constituting an adhesive composition suitable for dental use, comprising:
100 parts by weight of (A) at least one organic boron compound selected from the group consisting of a trialkyl boron, alkoxyalkyl boron, dialkyl borane, monocyclic or bicyclic compound having a boron atom as a hetero atom and partially oxidated trialkyl boron;
5 to 40 parts by weight of (B) an aprotic solvent having a boiling point of 50 to 120°C; and
0.2 to 5 parts by weight of (C) an alcohol having a boiling point of 60 to 180°C.

2. The polymerization initiator composition according to claim 1, wherein the aprotic solvent (B) is at least one solvent selected from the group consisting of an alkane, ketone, ether and ester.

3. The polymerization initiator composition according to claim 1 or 2, wherein the alcohol (C) is an alcohol having 4 or less carbon atoms.

4. The polymerization initiator composition according to any one of claims 1 to 3, wherein the organic boron compound (A) is partially oxidated tributyl boron, the aprotic solvent (B) is hexane, and the alcohol (C) is ethanol.

5. An adhesive composition suitable for dental or surgical use, comprising 30 to 100 parts by weight of (a) a polymerizable monomer, 0 to 70 parts by weight of (b) a (meth)acrylate polymer, and 1 to 20 parts by weight of (c) the polymerization initiator composition of any one of claims 1 to 4, the total of the polymerizable monomer (a) and the (meth)acrylate polymer (b) being 100 parts by weight.

6. The adhesive composition suitable for dental or surgical use according to claim 5, wherein the polymerizable monomer (a) is a (meth)acrylate or a combination of a (meth)acrylate and a polymerizable monomer having an acid group.

7. The adhesive composition suitable for dental or surgical use according to claim 5 or 6, wherein the (meth)acrylate polymer (b) is at least one polymer selected from the group consisting of a homopolymer of an alkyl (meth)acrylate, a copolymer of alkyl (meth)acrylates, a copolymer of an alkyl (meth)acrylate and another polymerizable monomer, a copolymer of an alkyl (meth)acrylate and an alkylene di(meth)acrylate, and a copolymer of an alkyl (meth)acrylate and a diene monomer.

## Patentansprüche

1. Polymerisationsinitiatorzusammensetzung für ein polymerisierbares Monomer, das eine Klebstoffzusammensetzung, die für die zahnärztliche Verwendung geeignet ist, bildet, umfassend:
100 Gewichtsteile von (A) wenigstens einer organischen Borverbindung, ausgewählt aus der Gruppe, bestehend aus einem Trialkylboran, einem Alkoxyalkylboran, einem Dialkylboran, einer monocyclischen oder bicyclischen Verbindung mit einem Boratom als ein Heteroatom und einem teilweise oxidierten Trialkylboran;
5 bis 40 Gewichtsteile von (B) einem aprotischen Lösungsmittel mit einem Siedepunkt von 50 bis 120°C und
0,2 bis 5 Gewichtsteile von (C) einem Alkohol mit einem Siedepunkt von 60 bis 180°C.

2. Polymerisationsinitiatorzusammensetzung gemäß Anspruch 1, wobei das aprotische Lösungsmittel (B) wenigstens ein Lösungsmittel, ausgewählt aus der Gruppe, bestehend aus einem Alkan, einem Keton, einem Ether und einem Ester, ist.

3. Polymerisationsinitiatorzusammensetzung gemäß Anspruch 1 oder 2, wobei der Alkohol (C) ein Alkohol mit 4 oder weniger Kohlenstoffatomen ist.

4. Polymerisationsinitiatorzusammensetzung gemäß einem der Ansprüche 1 bis 3, wobei die organische Borverbindung (A) teilweise oxidiertes Tributylboran ist, das aprotische Lösungsmittel (B) Hexan ist und der Alkohol (C) Ethanol ist.

5. Klebstoffzusammensetzung, die für die zahnärztliche oder chirurgische Verwendung geeignet ist, umfassend 30 bis 100 Gewichtsteile von (a) einem polymerisierbaren Monomer, 0 bis 70 Gewichtsteile von (b) einem (Meth)acrylatpolymer und 1 bis 20 Gewichtsteile von (c) der Polymerisationsinitiatorzusammensetzung nach einem der Ansprüche 1 bis 4, wobei die Gesamtheit des polymerisierbaren Monomers (a) und des (Meth)acrylatpolymers (b) 100 Gewichtsteile ist.

6. Klebstoffzusammensetzung, die für die zahnärztliche oder chirurgische Verwendung geeignet ist, gemäß Anspruch 5, wobei das polymerisierbare Monomer (a) ein (Meth)acrylat oder eine Kombination aus einem (Meth)acrylat und einem polymerisierbaren Monomer mit einer Säuregruppe ist.

7. Klebstoffzusammensetzung, die für die zahnärztliche oder chirurgische Verwendung geeignet ist, gemäß Anspruch 5 oder 6, wobei das (Meth)acrylatpolymer (b) wenigstens ein Polymer, ausgewählt aus der Gruppe, bestehend aus einem Homopolymer eines Alkyl(meth)acrylats, einem Copolymer von Alkyl(meth)acrylaten, einem Copolymer eines Alkyl(meth)acrylats und eines anderen polymerisierbaren Monomers, einem Copolymer eines Alkyl(meth)acrylats und eines Alkylendi(meth)acrylats und einem Copolymer eines Alkyl(meth)acrylats und eines Dienmonomers, ist.

## Revendications

1. Composition d'amorçage de polymérisation pour un monomère polymérisable constituant une composition adhésive appropriée pour un usage dentaire, comprenant :
100 parts en poids de (A) au moins un composé de bore organique sélectionné à partir du groupe constitué par un trialkylbore, un alcoxyalkylbore, un dialkylborane, un composé monocyclique ou bicyclique ayant un atome de bore comme hétéroatome et un trialkylbore partiellement oxydé ;
5 à 40 parts en poids de (B) un solvant aprotique ayant un point d'ébullition de 50 à 120 °C ; et
0,2 à 5 parts en poids de (C) un alcool ayant un point d'ébullition de 60 à 180 °C.

2. Composition d'amorçage de polymérisation selon la revendication 1, dans laquelle le solvant aprotique (B) est au moins un solvant sélectionné à partir du groupe constitué par un alcane, une cétone, un éther et un ester.

3. Composition d'amorçage de polymérisation selon la revendication 1 ou 2, dans laquelle l'alcool (C) est un alcool ayant 4 atomes de carbone ou moins.

4. Composition d'amorçage de polymérisation selon l'une quelconque des revendications 1 à 3, dans laquelle le composé de bore organique (A) est du tributylbore partiellement oxydé, le solvant aprotique (B) est de l'hexane et l'alcool (C) est de l'éthanol.

5. Composition adhésive appropriée pour un usage dentaire ou chirurgical, comprenant de 30 à 100 parts en poids de (a) un monomère polymérisable, de 0 à 70 parts en poids de (b) un polymère de (méth)acrylate, et de 1 à 20 parts en poids de (c) la composition d'amorçage de polymérisation selon l'une quelconque des revendications 1 à 4, le total du monomère polymérisable (a) et du polymère de (méth)acrylate (b) étant de 100 parts en poids.

6. Composition adhésive appropriée pour un usage dentaire ou chirurgical selon la revendication 5, dans laquelle le monomère polymérisable (a) est un (méth)acrylate ou une combinaison d'un (méth)acrylate et d'un monomère polymérisable ayant un groupe acide.

7. Composition adhésive appropriée pour un usage dentaire ou chirurgical selon la revendication 5 ou 6, dans laquelle le polymère de (méth)acrylate (b) est au moins un polymère sélectionné à partir du groupe constitué par un homopolymère d'un (méth)acrylate d'alkyle, un copolymère de (méth)acrylates d'alkyle, un copolymère d'un (méth)acrylate d'alkyle et d'un autre monomère polymérisable, un copolymère d'un (méth)acrylate d'alkyle et d'un di(méth)acrylate d'alkylène, et un copolymère d'un (méth)acrylate d'alkyle et d'un monomère diène.
